# EUROPEAN PATENT APPLICATION

(11) **EP 1 593 964 A1**
(43) Date of publication of application: **09.11.2005**
(21) Application number: 05252470.9
(22) Date of filing: 20.04.2005
(51) Int. Cl.: G01N 33/00, A61M 21/00, G11B 31/00, H04S 1/00, G02B 27/01

(54) **Biological sensor device and content playback method and apparatus**

(30) Priority: 07.05.2004 JP 2004138142
(71) Applicant: SONY CORPORATION, Tokyo 141-0001 (JP)
(72) Inventor: Katsuya, Shirai, Shinagawa-ku, Toyko 141-0001 (JP); Yoichiro, Sako, Shinagawa-ku, Toyko 141-0001 (JP); Toshiro, Terauchi, Shinagawa-ku, Toyko 141-0001 (JP); Makoto, Inoue, Shinagawa-ku, Toyko 141-0001 (JP); Masamichi, Asukai, Shinagawa-ku, Toyko 141-0001 (JP); Yasushi, Miyajima, Shinagawa-ku, Toyko 141-0001 (JP); Kenichi, Makino, Shinagawa-ku, Toyko 141-0001 (JP); Motoyuki, Takai, Shinagawa-ku, Toyko 141-0001 (JP)
(74) Representative: Nicholls, Michael John

(57) **Abstract**

A biological sensor device includes a sensor-mounting element worn on a portion from a front surface to a side surface of an upper part of a face of a user; and a plurality of biological sensors that is installed on the sensor-mounting element and that measures different types of biological conditions of the user.

## Description

The present invention contains subject matter related to Japanese Patent Application JP 2004-138142 filed in the Japanese Patent Office on May 7, 2004, the entire contents of which are incorporated herein by reference.

The present invention relates to biological sensor devices that acquire biological information by measuring biological conditions of users (conditions of living bodies) and to methods and apparatuses for playing back content, such as images and sound.

Due to the wide use of wearable computing, a procedure for measuring biological conditions of users using sensors installed on various accessories worn on the bodies of the users and for determining the degrees of fatigue, relaxation, and the like of the users in accordance with results of the measurement has been suggested.

For example, Japanese Unexamined Patent Application Publication No. 6-51902 discloses an information processing method using a light-emitting element and light-receiving element mounted on eyeglasses worn by an operator of office automation (OA) equipment. In this method, based on the fact that reflected light detected by the light-receiving element is reduced when the operator follows a screen of a display since light from the light-emitting element is projected to the eyes and on the fact that reflected light detected by the light-receiving element increases when the field of view of the operator moves outside the screen of the display since light from the light-emitting element is not incident on the eyes, the degree of fatigue of the operator is estimated. If it is determined that the degree of fatigue increases, a message, such as "Take a little break", or a desired image is displayed on the screen for the operator.

In addition, for example, Japanese Unexamined Patent Application Publication No. 10-76012 discloses a relaxation guidance apparatus and a biofeedback guidance apparatus. In paragraph 0140 and Fig. 43 of Japanese Unexamined Patent Application Publication No. 10-76012, a procedure for measuring the pulse rate of a user who receives autogenic training as relaxation training or who receives biofeedback treatment using a pulse wave sensor installed on the frames of eyeglasses worn by the user and for determining whether or not the degree of relaxation of the user is increasing in accordance with the change in measured values over time is described. If the degree of relaxation is increasing, the fact that the degree of relaxation is increasing is informed to the user by projection of light from a liquid crystal panel installed onto the eyeglass frames to the lenses of the glasses.

In the apparatus and method described in Japanese Unexamined Patent Application Publication No. 6-51902, the field of view (the position of the pupils) of the user is detected using optical sensors, such as a light-emitting element and a light-receiving element. Although the degree of fatigue of the user can be estimated, it is difficult, for example, to estimate and determine the psychological state of a user who views or listens to content, such as images or sound. Thus, it is difficult to use such an apparatus and method in the field of entertainment, such as content playback.

In addition, in the apparatus and method described in Japanese Unexamined Patent Application Publication No. 10-76012, although the pulse rate of the user can be measured using the pulse wave sensor, it is difficult, for example, to estimate and determine the psychological state of a user who views or listens to content, such as images or sound. Thus, it is also difficult to use such an apparatus and method in the field of entertainment, such as content playback.

It is desirable to provide a biological sensor device capable of determining the psychological state of a user who views or listens to content and a content playback method capable of controlling the playback state of the content in accordance with the psychological state of the user who views or listens to the content being played back and capable of playing back content most appropriate for the psychological state of the user.

According to an embodiment of the present invention, a biological sensor device includes a sensor-mounting element worn on a portion from a front surface to a side surface of an upper part of a face of a user; and a plurality of biological sensors that is installed on the sensor-mounting element and that measures different types of biological conditions of the user.

According to an embodiment of the present invention, a content playback method includes the steps of determining a psychological state of a user who views or listens to content by analyzing a plurality of different types of biological information of the user acquired by a plurality of biological sensors installed on a sensor-mounting element worn on a portion from a front surface to a side surface of an upper part of a face of the user; and controlling a playback state of the content in accordance with a result of determination performed in the determination step.

In the biological sensor device with the foregoing arrangement, a plurality of biological sensors for measuring different types of biological conditions, such as an electromyography sensor and a temperature sensor, a temperature sensor and a vibration sensor, or a vibration sensor and a blood flow sensor, is installed on a sensor-mounting element worn on a portion from the front surface to a side surface of the upper part of the face so as to easily detect various biological conditions of a user. Thus, by analyzing the plurality of different types of biological conditions acquired by the plurality of biological sensors, the psychological state of the user who views or listens to content can be estimated and determined.

In the content playback method with the foregoing arrangement, the psychological state of a user who views or listens to content can be determined by using the biological sensor device. In accordance with a result of the determination, the playback state of the content, such as the speed of playing back the content, the contrast of a picture, the brightness of the picture, the color of the picture, the rhythm of music, a frequency characteristic of sound, or the volume of the output sound, can be controlled. Thus, the content can be played back most appropriately for the psychological state of the user.

As described above, a biological sensor device capable of determining the psychological state of a user who views or listens to content is provided. In addition, a content playback method capable of controlling the playback state of the content in accordance with the psychological state of the user who views or listens to the content being played back and capable of playing back content most appropriate for the psychological state of the user is provided.

The invention will now be described by way of non-limiting example with reference to the accompanying drawings, in which:
Fig. 1 illustrates a biological sensor device according to an embodiment of the present invention;
Fig. 2 is a block diagram showing a content playback apparatus according to an embodiment of the present invention;
Fig. 3 is a flowchart of a playback process performed by a controller of the content playback apparatus;
Fig. 4 illustrates a content playback system according to a modification of the present invention;
Fig. 5 illustrates a biological sensor device according to a modification of the present invention; and
Fig. 6 illustrates a content playback system according to another modification of the present invention.

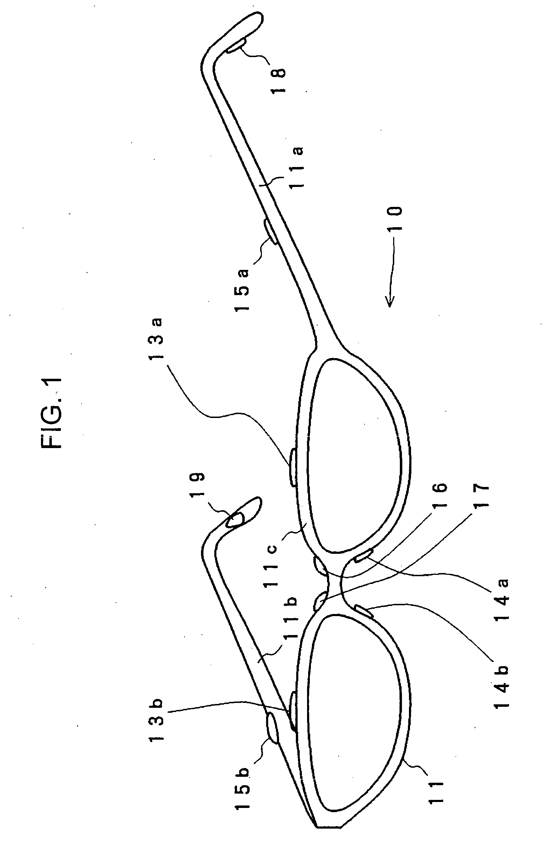

Fig. 1 illustrates a biological sensor device 10 according to an embodiment of the present invention. A plurality of biological sensors is installed on a sensor-mounting element having the shape of eyeglass frames.

More specifically, a sensor-mounting element 11 of the biological sensor device 10 includes a front temple subelement 11c and left and right temple subelements 11a and 11b. Electromyography sensors 13 (13a and 13b) are installed on the upper part of the front temple subelement 11c. Temperature sensors 14 (14a and 14b), a vibration sensor 16, and an angle sensor 17 are installed at the center of the front temple subelement 11c. Temperature sensors 15 (15a and 15b) are installed on the left and right temple subelements 11a and 11b, respectively. A blood flow sensor 18 is installed on the leading end of the left temple subelement 11a, and a pulse wave sensor 19 is installed on the leading end of the right temple subelement 11b.

Each of the electromyography sensors 13a and 13b is a strain sensor or a piezoelectric sensor. The electromyography sensors 13a and 13b measure the myoelectric potential of the eyebrows. When human beings are happy, the myoelectric potential is low. When human beings are sad, angry, or scared, the myoelectric potential is high. Thus, the countenance of a user whose myoelectric potential is measured can be estimated in accordance with the values measured by the electromyography sensors 13a and 13b.

The temperature sensors 14a and 14b measure the temperature of the bridge of the nose of the user. The temperature sensors 15a and 15b measure the temperature of the temples of the user. When human beings feel uncomfortable, the temperature of the bridge of the nose is low. Thus, a feeling of comfort or discomfort of the user can be estimated in accordance with the difference (absolute value) between the values measured by the temperature sensors 14a and 14b and the values measured by the temperature sensors 15a and 15b. When the difference is small, it is determined that the user feels comfortable. When the difference is large, it is determined that the user feels uncomfortable.

The vibration sensor 16 measures the vibration of an area between the eyebrows of the user to detect blinking of the user. The angle sensor 17 detects motion of the whole body of the user or motion of the head of the user to the left and right. When human beings are concentrating on something, the blink rate and the rate of body motion are small. When human beings are not concentrating on something, the blink rate and the rate of body motion are large. Thus, the degree of concentration of the user can be estimated in accordance with the value measured by the vibration sensor 16 or the angle sensor 17.

The blood flow sensor 18 measures the volume of blood flow at one ear of the user. The pulse wave sensor 19 measures the pulse rate at the other ear of the user. When human beings feel comfortable, the volume of peripheral blood flow increases and the pulse rate decreases. When human beings feel uncomfortable, the volume of peripheral blood flow decreases and the pulse rate increases. Thus, the feeling of comfort or discomfort of the user can be estimated in accordance with a change in the value measured by the blood flow sensor 18 or the pulse wave sensor 19.

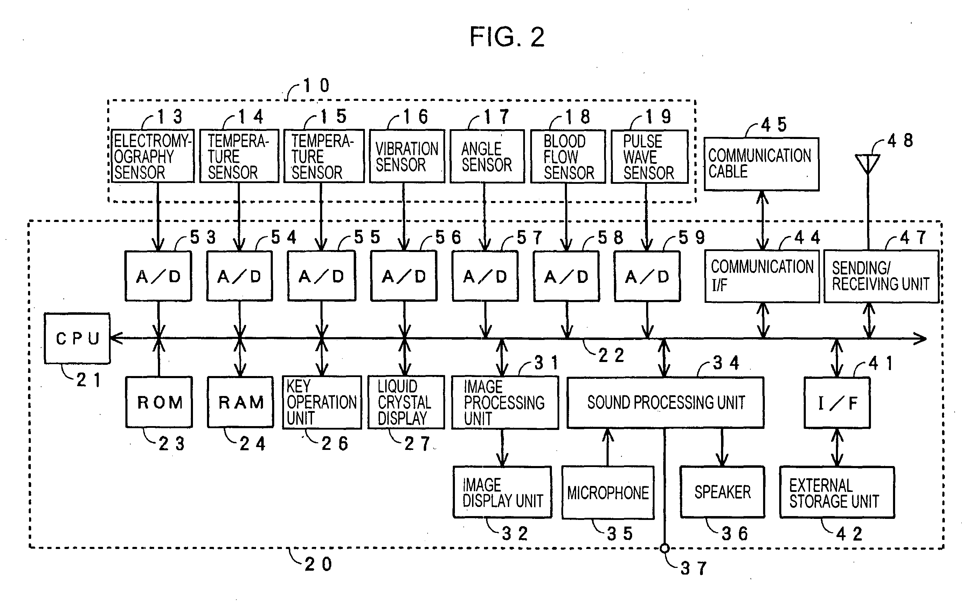

Fig. 2 is a block diagram showing a content playback apparatus 20 according to an embodiment of the present invention using the biological sensor device 10 when images and sound are played back as content.

The content playback apparatus 20 includes a central processing unit (CPU) 21. A read-only memory (ROM) 23 in which a program performed by the CPU 21, necessary fixed data, and the like are written, a random-access memory (RAM) 24 into which the program and the data are loaded, a key operation unit 26, and a liquid crystal display 27 are connected to a bus 22 for the CPU 21.

The user who views and listens to the content can select, by a selecting operation using the key operation unit 26, a playback mode in the content playback apparatus 20 between a playback control mode in which the content playback state is controlled using the biological sensor device 10 and a playback non-control mode in which the content playback state is not controlled and in which the playback state is maintained at a predetermined content playback mode. The liquid crystal display 27 displays operating conditions and the operation state of the content playback apparatus 20.

Furthermore, an image display unit 32 is connected to the bus 22 via an image processing unit 31, and an external output terminal 37 connected to a microphone 35, a speaker 36, a headphone, and the like is connected to the bus 22 via a sound processing unit 34.

An external storage unit 42, such as a memory card or an optical disk, is connected to the bus 22 via an interface 41. A communication cable 45 is connected to the bus 22 via a communication interface 44. An antenna 48 is connected to the bus 22 via a sending/receiving unit 47.

Data of images and sound to be played back is stored in the external storage unit 42. The data of the images and sound to be played back is captured in the content playback apparatus 20 from an external apparatus via the communication cable 45 or received as broadcast signals by the content playback apparatus 20 via the antenna 48.

In this example, the electromyography sensors 13 (13a and 13b), the temperature sensors 14 (14a and 14b), the temperature sensors 15 (15a and 15b), the vibration sensor 16, the angle sensor 17, the blood flow sensor 18, and the pulse wave sensor 19 are connected to the bus 22 via analog-to-digital (A/D) converters 53, 54, 55, 56, 57, 58, and 59, respectively.

The electromyography sensors 13 are electromyography sensors 13a and 13b. The A/D converters 53 are two A/D converters converting analog signals output from the electromyography sensors 13a and 13b into digital data. A similar arrangement is applied to the temperature sensors 14 and 15.

In this example, when the content playback apparatus 20 plays back an image and sound by reading data of the image and sound recorded in the external storage unit 42, by capturing data of the image and sound in the content playback apparatus 20 from an external apparatus via the communication cable 45, or by receiving data of the image and sound as broadcast signals at the content playback apparatus 20 via the antenna 48, if the playback control mode is set, the CPU 21 analyzes biological information serving as an output of each biological sensor of the biological sensor device 10, in accordance with a playback processing program described below. The CPU 21 makes a comprehensive evaluation of the analysis results, and determines the psychological state of the user who wears the biological sensor device 10 and who views and listens to the image and sound. The CPU 21 controls the playback state of the image and sound in accordance with the determination result.

More specifically, as described above, the countenance of the user is estimated in accordance with the result measured by the electromyography sensor 13a or 13b, the feeling of comfort or discomfort of the user is estimated in accordance with the difference between the value measured by the temperature sensor 14a or 14b and the value measured by the temperature sensor 15a or 15b. Also, the degree of concentration of the user is estimated in accordance with the value measured by the vibration sensor 16 or the angle sensor 17, and the feeling of comfort or discomfort of the user is estimated in accordance with a change in the value measured by the blood flow sensor 18 or the pulse wave sensor 19. By comparing these estimation results with predetermined criteria, it is finally determined whether or not the user feels comfortable.

In brief, when it is estimated that the user is happy, pleasant, and concentrating on something, it is determined that the user feels comfortable. In contrast, when it is estimated that the user is sad, angry, or scared and that the user feels discomfort and is not concentrating on something (inattentive), it is determined that the user feels uncomfortable. For an intermediate case, determination is performed in accordance with the predetermined criteria.

When it is determined that the user feels comfortable, the image and sound are played back in the current state. When it is determined that the user feels uncomfortable, the playback state of the image and sound is changed in the direction that reduces a stimulus to the user so that the user feels more comfortable.

More specifically, when it is determined that the user feels uncomfortable, the speed of playing back the image and sound is reduced, the contrast or brightness of a picture is reduced, the hue of the picture is changed to a quiet hue, the color saturation of the picture is reduced, the rhythm of music is reduced, the frequency characteristics of sound, such as music, are increased for the midrange and reduced for the bass and treble ranges, or the volume of the output sound, such as music, is reduced.

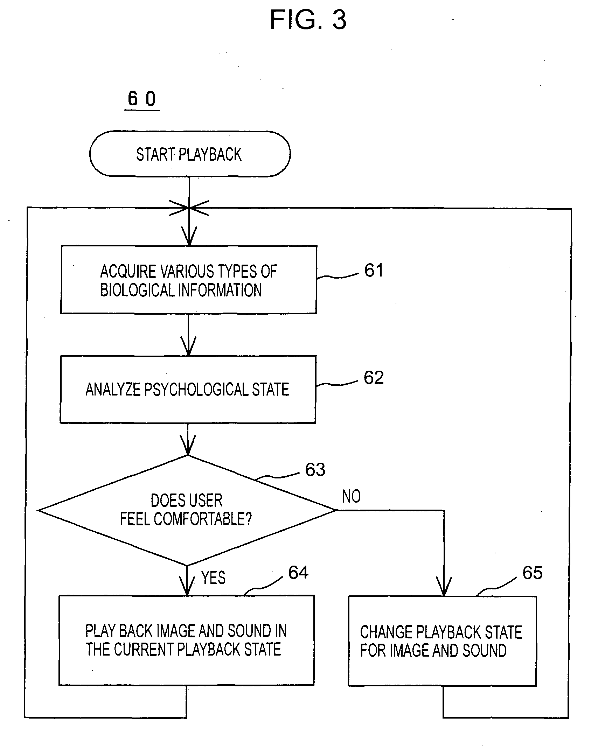

Fig. 3 is a flowchart of a playback process performed by the CPU 21 in the playback control mode. In a playback processing program 60 in this example, the user wears the biological sensor device 10 to start playing back content. In step S61, the CPU 21 acquires various types of biological information of the user using the various biological sensors of the biological sensor device 10. In step S62, the CPU 21 analyzes the psychological state of the user in accordance with the various types of acquired biological information. In step S63, the CPU 21 determines whether or not the user feels comfortable in accordance with the analysis results.

If the CPU 21 determines that the user feels comfortable in step S63, the process proceeds to step S64 to play back an image and sound in the current playback state. Then, the process returns to step S61. In contrast, if the CPU 21 determines that the user feels uncomfortable in step S63, the process proceeds to step S65 to change the playback state of the image and sound as described above. Then, the process returns to step S61.

By repeating this process, the user can view images and listen to sounds in a state in which the user feels comfortable.

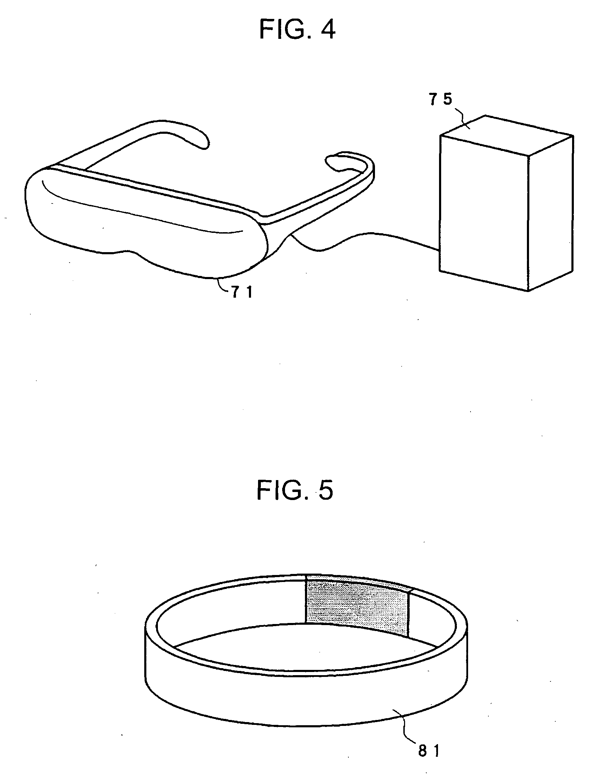

As shown in Fig. 4, a content playback system may include an image and sound playback apparatus 75 and a head-mount display 71 used for viewing images and listening to sounds played back by the sound playback apparatus 75. In this case, various biological sensors may be installed on the head-mount display 71 so as to use the head-mount display 71 as a biological sensor device. With this arrangement, the user need not wear the biological sensor device 10 shown in Fig. 1, independent of the head-mount display 71.

As shown in Fig. 5, a head-band biological sensor device 81 on which various biological sensors are installed, instead of a biological sensor device having the shape of eyeglass frames, may be used.

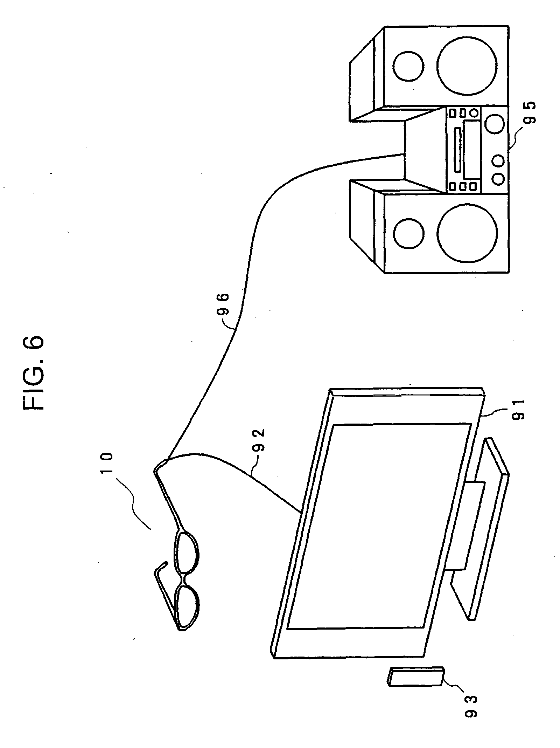

In addition, as shown in Fig. 6, a content playback system may include the biological sensor device 10, an image playback apparatus 91 including a remote-control transmitter 93, and a sound playback apparatus 95. In this case, the biological sensor device 10, the image playback apparatus 91, and the sound playback apparatus 95 may be connected to each other using cables 92 and 96 or radio communication.

Although a case where images and sound are played back as content has been described, the present invention is not necessarily limited to this. The present invention is also applicable to a case were only information that can be visually sensed, such as images (pictures), is played back or a case where only information that can be acoustically sensed, such as sound (music), is played back.

In addition, biological sensors that are installed on a sensor-mounting element and that measure biological conditions of human beings are not necessarily limited to an electromyography sensor, a temperature sensor, a vibration sensor for measuring blinking, an angle sensor for measuring body motion, a blood flow sensor, and a pulse wave sensor. Sensors for measuring skin perspiration, skin resistance, and microvibration (MV) may be used, as long as they can measure biological conditions of human beings at a portion from the front surface to a side surface of the upper part of the face.

It should be understood by those skilled in the art that various modifications, combinations, sub-combinations and alterations may occur depending on design requirements and other factors insofar as they are within the scope of the appended claims or the equivalents thereof.

## Claims

1. A biological sensor device comprising:
a sensor-mounting element worn on a portion from a front surface to a side surface of an upper part of a face of a user; and
a plurality of biological sensors that is installed on the sensor-mounting element and that measures different types of biological conditions of the user.

2. The biological sensor device according to Claim 1, wherein the sensor-mounting element has a shape of eyeglass frames.

3. A content playback method comprising the steps of:
determining a psychological state of a user who views or listens to content by analyzing a plurality of different types of biological information of the user acquired by a plurality of biological sensors installed on a sensor-mounting element worn on a portion from a front surface to a side surface of an upper part of a face of the user; and
controlling a playback state of the content in accordance with a result of determination performed in the determination step.

4. The content playback method according to Claim 3, wherein in the controlling step, the speed of playing back the content, the contrast of a picture, the brightness of the picture, the color of the picture, the rhythm of music, a frequency characteristic of sound, or the volume of the output sound is controlled as the playback state of the content.

5. A content playback apparatus comprising:
content playback means for playing back content by reading content data from a recording medium or by receiving the content data from an external apparatus;
determination means for determining a psychological state of a user who views or listens to the content being played back by the content playback means by analyzing a plurality of different types of biological information of the user acquired by a plurality of biological sensors installed on a sensor-mounting element worn on a portion from a front surface to a side surface of an upper part of a face of the user; and
control means for controlling a playback state of the content played back by the content playback means in accordance with a result of determination performed by the determination means.

6. The content playback apparatus according to Claim 5, wherein the control means controls the speed of playing back the content, the contrast of a picture, the brightness of the picture, the color of the picture, the rhythm of music, a frequency characteristic of sound, or the volume of the output sound as the playback state of the content.

7. The content playback apparatus according to Claim 5 or 6, further comprising mode setting means for selectively setting on and off the control of the playback state of the content performed by the control means.

8. A content playback apparatus comprising:
a content playback unit playing back content by reading content data from a recording medium or by receiving the content data from an external apparatus;
a determination unit determining a psychological state of a user who views or listens to the content being played back by the content playback unit by analyzing a plurality of different types of biological information of the user acquired by a plurality of biological sensors installed on a sensor-mounting element worn on a portion from a front surface to a side surface of an upper part of a face of the user; and
a controller controlling a playback state of the content played back by the content playback unit in accordance with a result of determination performed by the determination unit.
